# EUROPEAN PATENT APPLICATION

(11) **EP 4 582 683 A1**
(43) Date of publication of application: **09.07.2025**
(21) Application number: 23858476.7
(22) Date of filing: 01.09.2023
(51) Int. Cl.: F02D 19/08, G01N 27/22, G01N 33/22

(54) **FUEL SENSOR AND SENSING METHOD**

(30) Priority: 02.09.2022 BR 102022017734
(71) Applicant: Marelli Sistemas Automotivos Industria e Comercio Brasil Ltda, 13184654 Hortolândia (BR)
(72) Inventor: GAVIOLI, Vagner Eduardo, 13478-713 Americana (BR); RIEDO, Sandro, 13257-484 ITATIBA (BR); DOS SANTOS, Paulo Henrique, 13060-858 CAMPINAS (BR); DUTRA E SILVA, Gabriel Henrique, 13185-342 Hortolândia (BR); BORRASCHI ANTONIO, Rodrigo, 13467-662 Americana (BR); ALVES SAMPAIO E SILVA, Alexandre, 13901-093 Amparo (BR); WINDLIN, Fernando Luiz, 13211-616 Jundiaí (BR); DOS SANTOS ARAUJO, Gilson Fernando, 13175-658 Sumaré (BR); MAYER ALEGRE, Guilherme Henrique, 13212-070 Jundiaí (BR); PEREIRA BESSA, Diogo, 13056-514 Campinas (BR)
(74) Representative: Novagraaf International SA
(86) International application number: PCT/BR2023/050291
(87) International publication number: WO 2024/044831

(57) **Abstract**

A fuel sensor (1) for detecting the percentage of ethanol in a gasoline and ethanol fuel mixture is described. The fuel sensor comprises a casing (2) that defines a seat (9) for a PCB (8), while the casing (2) comprises, externally, tabs (3) that project from opposite sides, an electrical connection terminal (6) and two hydraulic connection terminals (4,5) to go to the fuel reservoir tube and the fuel train tube. The fuel sensor further comprises a sensing element (10) in the form of an outside cylindrical cover (11) wrapped around an internal cylindrical core (12). The sensing element (10) is attached to the casing (2) from a cylindrical support (14), while the cover (11) is electrically connected to the PCB board (8) from a first contact pin (15), and the core (12) is electrically connected to the PCB board (8) from a second contact pin (16), said contact pins (15, 16) passing through the cylindrical support (14) and being supported and held in position by a plastic substrate (2) fixed inside the casing (20) seat (9).

## Description

The present invention relates to a fuel sensor, such as a sensor to identify the percentage in a mixture of two fuels.

### STATE OF THE ART

As is widely known by those skilled in the art, mixing fuels is one solution used in Brazil and other parts of the world, too. Specifically, the use of fuel in the form of a mixture of alcohol from any possible source, especially renewable sources, and gasoline, especially ethanol and gasoline, offers a series of advantages to consumers, such as better control of the cost of fuel per kilometer driven. Furthermore, consumers with enhanced technical knowledge are also able to manage the difference in performance of internal combustion engines (ICE) for each of these fuels individually. In this regard, and due to the chemical differences between ethanol and gasoline, especially in terms of the size of the chemical chain (the carbon chain of gasoline is longer and more varied than that of ethanol), burning each of these fuels embodies specificities that lead to variations in the respective compression rate and torque created by ICE. It is no less important to remember that, because of these same chemical differences, specifically in the respective carbon chains, the amount of oxygen required to burn a given volume of gasoline is different from the amount of oxygen required to burn the same volume of ethanol.

Regardless of the advantages mentioned above, the use of variable fuel mixtures or, in other words, where the percent of each individual fuel in the mixture is freely selected by the driver, varies as a function of the fuel supplied to the vehicle tank, and leads to management issues that go beyond the normal ICE control as a function of the instantaneous conditions of the vehicle. Accordingly, since the driver requires a certain amount of torque (by pressing on the accelerator and thus releasing air flow), the ECU (Electronic Control Unit that controls the engine management system of a vehicle) determines the amount of fuel in the form of injection time as a function of the traditional variables (input air flow, temperature, engine rotation, etc.), but also as a function of the characteristics of the fuel (or mixture of fuels) to be burned or, in practical terms, the A/F ratio (air/fuel ratio) determined by the respective percentages of the individual fuels that make up the fuel mixture in use.

Some ways to determine the A/F ratio for mixing fuels being used are known in the state of the art. These can be split into two groups:
Group 1 - calculated from the characteristics of the gases generated by burning the fuel. In this case, the result of the combustion of a given mass of fuel comprised of an unknown mixture is monitored as a function of the result obtained (composition of the exhaust gases via an oxygen sensor, torque, etc.), and the A/F value is duly adjusted to obtain the ideal fuel burn. The main disadvantages of these methods consist of the time required to obtain the exact value of the A/F ratio for the mixture that is being burned in terms of the engine cycle, and mostly, the constant processing of this data, consuming part of the ECU's finite processing capacity. These factors add to the criticalities imposed by increased combustion pressure in supercharged engines with direct injection in the fuel chamber (DI - direct injection).
Group 2 - using fuel sensors, generally arrayed in the fuel feed line between the fuel reservoir and the injector rail, and that can eliminate the need for any type of additional processing to obtain the A/F ratio, as the analog or digital value provided by the sensor indicates the fuel composition, which is directly tied to an accurate A/F value. In the sensors currently available in the market, the percent composition of the fuel is determined by measuring capacitance, even though there are publications mentioning other mechanisms/properties to obtain percent fuel composition.

Another function of said fuel sensor is to determine, for instance via capacitance, the percent water in the mixture, which compromises the lifetime of the injection system components.

Notwithstanding said advantage in terms of ECU processing savings, fuel sensors are not free of drawbacks either. Among them, the more relevant hurdles for massive deployment of this line of technical solution are the manufacturing cost and the accuracy of the readings.

In fact, determining the percent ethanol and water exclusively by measuring the capacitance of the fuel mixture has historically proven to be an inaccurate solution, as the curve that defines it is not a well-behaved straight line, but has regions of different slopes, thus adversely affecting the accuracy of the indication in certain ranges of ethanol and water concentration.

Document US 2008/295574 or BRPI0801181 generically teaches that the determination of the water content in a fuel mixture enables improved control of a vehicle engine. Nevertheless, no means of realization for a sensor of this type is presented.

Document US 10018613 teaches an LCR resonant circuitry-based sensing device. Electrodes are used to apply an electrical stimulus to the fluid being analyzed, while the return signal is analyzed by processors to determine the composition of the fluid. In particular, at least one processor is used to determine the percent water in the fluid based on processing the resonant impedance spectral response. Even though this device is capable of determining, via processing, the percent water (among others) in a fluid, it may pose risks if the mixture is not inert, as an electrical current must go through the liquid.

Document US 5150683 teaches a method and sensor for determining the respective percentages in a mixture of two fluids with substantially different dielectric properties, such as mixtures of gasoline and alcohol, or fuel oil and water. An inductive coil element immersed in the mixture exhibits a capacitance level that is directly proportional to the dielectric constant of the fluid mixture surrounding the coil, which in turn is directly proportional to the percentage of each fluid in the mixture. The loops of wire in the coil act as equivalent electrodes, thus creating a capacitance between each loop. A circuit converts the signal into frequency (generated by an amplifier that oscillates at the resonant frequency of the LC circuit of the coil) and sends it to the engine control to promote the necessary adjustments in ignition time.

Document JPH 0443835 teaches an alcohol sensor in a fuel mixture where the temperature of the mixture and the amount of water in the mixture are taken into consideration to obtain more accurate alcohol quantification. These parameters are used to compensate for the percent alcohol detected by a fuel sensor. The changes in oscillation frequency are analyzed as [they are] due to the percent alcohol and temperature of the mixture, among others, through the electrostatic capacity of the mixture passing across two sensor electrodes. A table inserted into the computing means is used to offset the percent alcohol.

### OBJECTIVES OF THE INVENTION

Accordingly, the first objective of the invention is a sensor device for detecting the percentage of a fuel mixture in flow, capable of overcoming the drawbacks of the art.

Another objective of the present invention is a fuel sensor capable of determining the percent ethanol in a fuel mixture comprising ethanol and gasoline.

This and other objectives of the present invention are achieved and fully satisfied with a fuel sensor as defined in claim 1.

### BRIEF DESCRIPTION OF THE DRAWINGS

The object of the present invention will be better understood from the following detailed description of a preferred embodiment of the invention, which is done with the support of the accompanying drawings, used as illustration and not meant to be limiting, wherein:

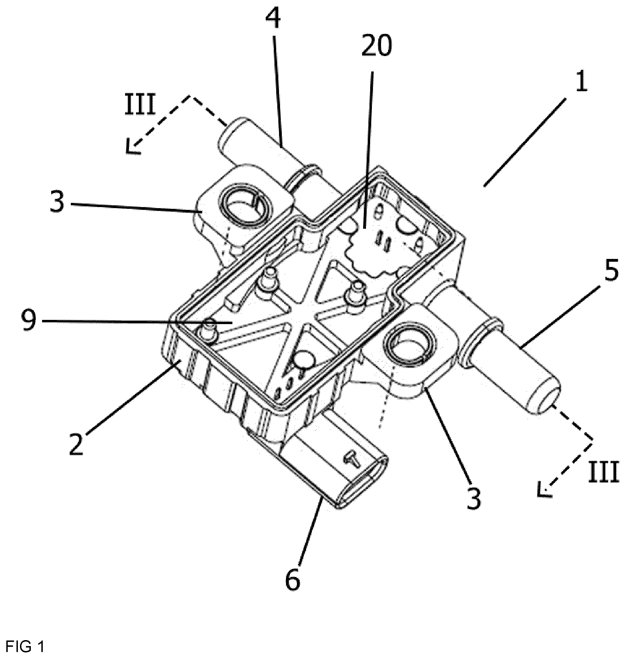

- Figure 1 is a perspective view of a fuel sensor according to the invention;
- Figure 2 is a cross-section of the fuel sensor according to cut II-III of figure 1;
- Figure 3 is a schematic perspective view showing the electrical connections of the measuring cover (outside tube) and core (inner tube) through the respective contact pins;
- Figure 4 is a cross-sectional view of the sensor tubes according to cut IV-IV of figure 3;
- Figure 5 is an expanded top view of the fuel sensor; and
- Figures 6A and 6B are expanded lower views of the fuel sensor, showing the two assembly steps for the parts comprising the sensor.

According to the illustrations described above, with 1, throughout, there is indicated a fuel sensor in accordance with the present invention. The fuel sensor, also designatable as an ethanol sensor, is basically defined by a paralleleliped casing 2 that is empty inside, and that defines a seat 9 capable of receiving a circuit board, or PCB 8, which receives the connections with sensing element 10, with connector 6, and holds the electronic components used to process the signals received from the sensing element, sending a processed signal to the ECU (not shown) of a vehicle (not shown) via connector 6. Opposite sides of casing 2 will be fitted with tabs 3 that are an integral part of said casing for attaching it to an appropriate substrate of the vehicle, such as the chassis or, alternatively, the ICE. For this, an oblong bushing 19 is inserted into each through-hole of the tabs 3, each of which is preferably arrayed 90° relative to the other.

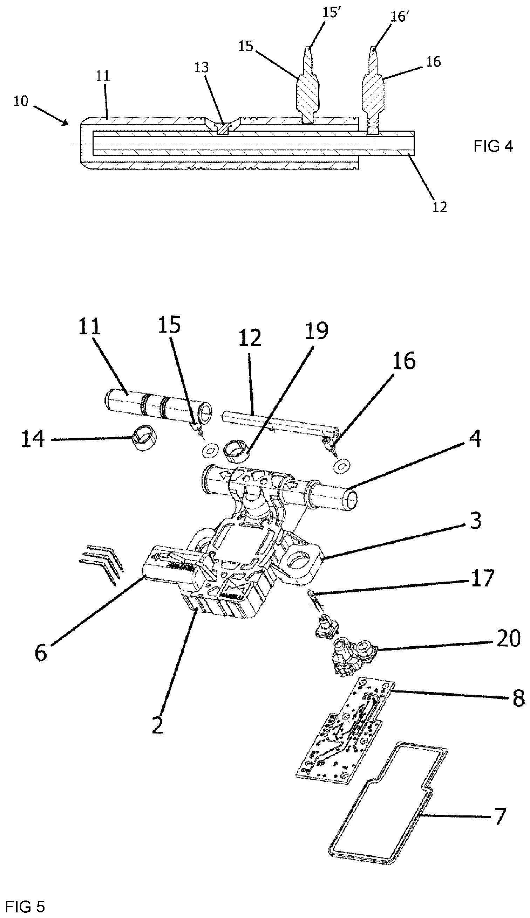

Two connection terminals, 4 and 5, project opposite each other from the upper sides, to be used respectively to connect to a tube (not shown) from the fuel reservoir and the tube (not shown) leading to the fuel train that feeds the ICE. More specifically, from the lower portion of casing 2 (see in particular figure 5), a cylindrical support 14 integrated into said casing is foreseen, capable of keeping said sensing element 10 attached internally. A first projecting extremity of cylindrical support 14 defines the input terminal 4 for the tube coming from the fuel reservoir.

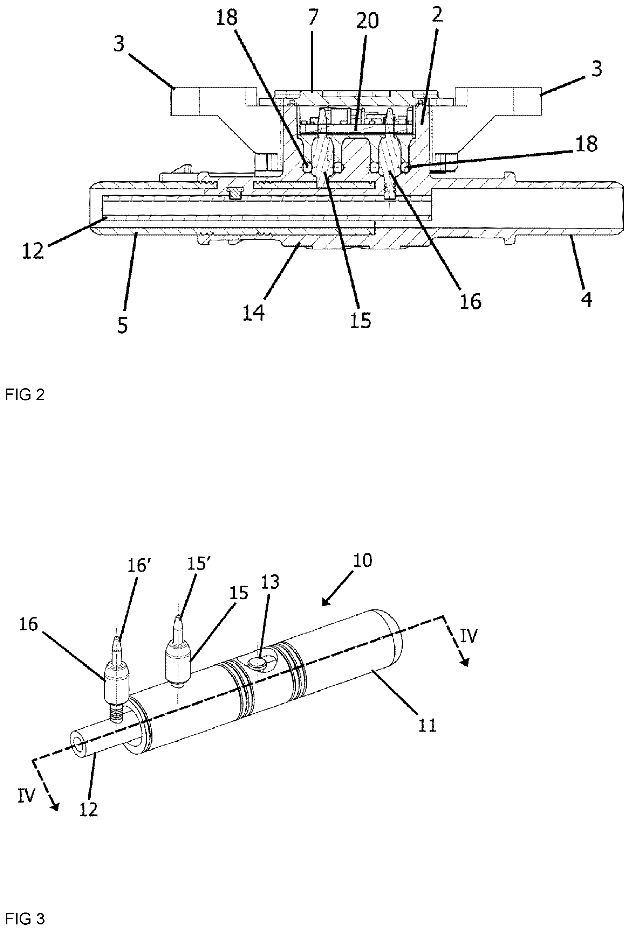

Said sensing element 10 is mechanically attached to the inside of the cylindrical support 14, and hydraulically insulated from the inside of the casing 2. Said sensing element 10, as illustrated in figures 3 and 4, is defined by an external cover 11 that concentrically receives an internal core 12. The fuel coming from the input terminal 4 goes through the sensing element 10 in the cylindrical element externally delimited by the cover 11 and internally delimited by the core 12. The outer cover 11 extends beyond the cylindrical support 14 and defines the output terminal 5, which can be coupled by said fuel train feed tube.

Cover 11 and core 12 are attached in the central region of sensing element 10 using lock 13 working with contact pins 15, 16 described ahead. An NTC-type temperature sensor 17 (see figure 5) is also foreseen, capable of determining the temperature of a fuel mixture in the region of sensing element 10, where the physical characteristics of the fuel mixture are collected.

As better illustrated in figures 2 to 4, cover 11 and core 12 have respective electrical contact pins 16, 15 for the core and cover to the PCB board 8. Each of said contact pins 15, 16 are of a specific shape capable of being connected, for instance by welding, to PCB 8. More particularly, each of the contact pins has a cylindrical body with an upper projection 15', 16' of smaller diameter, capable of penetrating PCB 8 and being welded thereto, the lower part may be mechanically and electrically connected to respective cover 11 and core 12. Clearly, said contact pins 15, 16 are electrically insulated from each other by the insulating material of which the casing 2 is fabricated, among others. Hydraulic insulation of each of the contact pins 15, 16 is performed by sealing rings 18, or O-rings, that go around the cylindrical body of each of the contact pins, thus keeping the fuel flowing inside the sensing element from penetrating seat 9 of casing 2, as shown in figure 2.

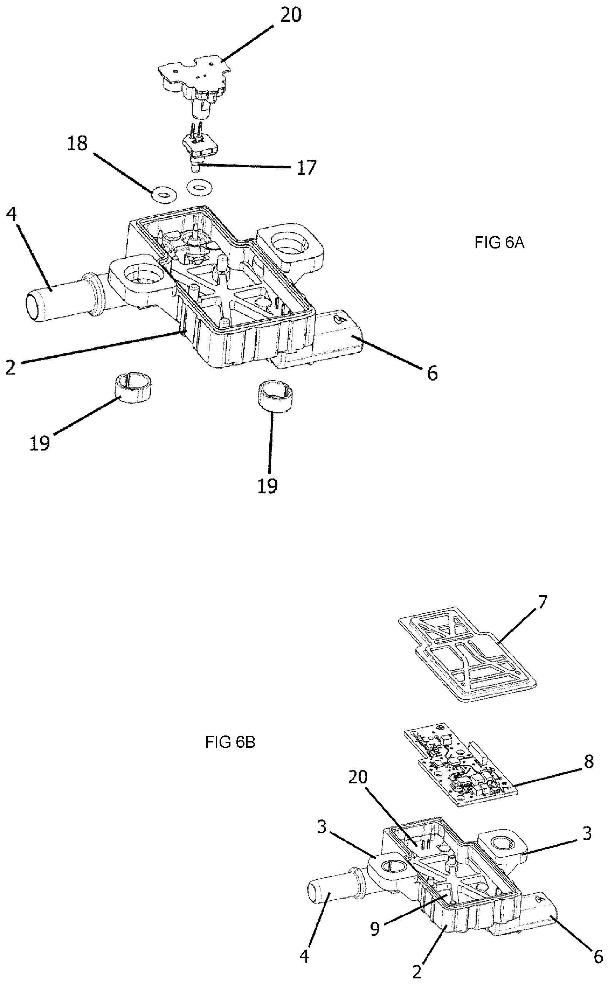

Said PCB board 8, as illustrated in figures 5 and 6B, shows a preferred conformation preferably cooperating with said seat 9 defined inside the casing 2, and further includes processing components such as a microprocessor or the like, capable of receiving the signals from the physical characteristics measured by the sensing element, and read the temperature provided by the temperature sensor NTC 17, and other discrete components. In particular, the discrete components installed in PCB 8 define an RC circuit that works in parallel with sensing element 10, in such a way as to excite/feed sensing element 10 and recover the voltage measurements from sensing element 10 immersed in the fuel flow, as per the technique known to the art.

The microprocessor can be a general use processor or a dedicated processing unit programmed so as to perform functions predetermined by the mode of action of the fuel sensor device 1. The microprocessor may further be comprised of one or more processors, where in the case of more than one processor each processor is responsible for performing one or more determined calculations. As used herein, the term "microprocessor" refers to processors in general, central processing units (CPU), application-specific integrated circuits (ASIC), logical circuits, and/or other circuits or processors capable of executing the functions according to the method of the present invention.

Regarding the assembly of the sensor 1, a casing 2, preferably made of engineering plastic, has overinjected (specifically by the cylindrical support 14) metal tubes (cover 11 and core 12), from which the data to determine the composition of the fuel mixture is collected. Additionally, and to offset and analyze the temperature of the fuel, an NTC temperature sensor 17 is housed in a plastic substrate 20, thus ensuring the correct positioning of the NTC sensor next to the metal wall of cover 11. More specifically, said plastic substrate 20 (see specifically figures 6A and 2), is a shaped plate capable of fitting into the inside of seat 9, in a position overlapping the cylindrical support 14. Said plastic substrate 20 supports and positions said temperature sensor 17, and also supports and keeps the contact pins 15, 16 in position from their respective upper projections 15' and 16', which penetrate the respective through-holes (not shown) of plastic substrate 20 so as to contact and be welded to the PCB 8, later inserted and fixed to the seat 9 of the casing 2 (see figure 6B).

Following insertion and positioning of the internal components of the casing 2 in order, a lid 7 is joined to the casing 2, preferably by welding or a similar process so as to ensure hermetic closing of the casing 2, and thus of the electro-electronic components located inside said casing 2.

Regarding the operation of said sensor 1, said physical characteristics are measured from the microprocessor injection of a signal of a given frequency, preferably using different frequencies for distinct physical characteristics. The return of the injected signal is split via a peak detector circuit and then processed according to the methodology of the present invention in the form of program instructions previously inserted into the microprocessor.

Finally, the processor collects information on the physical characteristics of the fuel mixture. Said values are then processed not in absolute terms, but as a function of the variable state, the temperature of the fuel mixture, so as to enhance the value of percent ethanol obtained in said fuel mixture.

Once said values for the physical characteristics for a given temperature measured are captured and processed, the microprocessor calculates the corresponding value for the ethanol portion of the mixture in a known manner.

This methodology enables determining a value for the percentage of ethanol and water in the fuel mixture with a high degree of accuracy, resulting in better adjustment of the ICE (internal combustion engine) operating conditions by the ECU (engine electronic control unit).

Furthermore, the cost to manufacture the sensor (1) as described is relatively lower compared to the sensors currently available in the market, enabling the large scale implementation thereof.

### EMBODIMENTS

A fuel sensor, in particular for detecting the percent ethanol in a fuel mixture comprised of gasoline and ethanol, said fuel sensor (1) comprising paralleleliped casing (2) that internally defines a seat (9) for a circuit board PCB (8), wherein the casing (2) contains external tabs (3) projecting from opposite sides, an electrical connection terminal (6), and two hydraulic connection terminals (4,5), one opposite the other and destined to be respectively attached to a tube coming from the fuel reservoir and a tube going to the fuel rail, said fuel sensor (1) further comprising a sensing element (10) in the form of an outer cylindrical cover (11) wrapped around an internal cylindrical core (12), and by said sensing element (10) to be attached to the casing (2) from a cylindrical support (14); sensing element (10) cover (11) being electrically connected to PCB board (8) from a first contact pin (15) and the core (12) being electrically connected to PCB board (8) using a second contact pin (16), said contact pins (15,16) going through cylindrical support (14), and supported and held in position by a plastic substrate (20) fixed inside the seat (9) and casing (2).

A fuel sensor, wherein the first contact pin (15) comprises a cylindrical body provided with a first upper cylindrical projection (15'), said first upper projection (15') being of a smaller diameter and able to penetrate a through-hole of the plastic substrate (20) and a respective through-hole of PCB board (8) and being welded thereto, and wherein the lower part of the cylindrical body of the first contact pin (15) is mechanically and electrically connected to the respective cover (11) of sensing element (10).

A fuel sensor, wherein the second contact pin (16) comprises a cylindrical body provided with a second upper cylindrical projection (16'), said second upper projection (16') being of a smaller diameter and able to penetrate a through-hole of the plastic substrate (20) and a respective through-hole of PCB board (8) and being welded thereto, and wherein the lower part of the cylindrical body of the second contact pin (16) is mechanically and electrically connected to the respective core (12) of sensing element (10).

A fuel sensor, wherein each of the first and second contact pins (15, 16) comprises the respective sealing rings (18) or O-rings going around the cylindrical body of each of said contact pins.

A fuel sensor, wherein the cylindrical support (14) is overinjected around the sensing element (10).

A fuel sensor, wherein the relative attachment between cover (11) and core (12) is performed in the central region of the sensing element (10) with a lock (13), attachment being achieved in cooperation with contact pins (15, 16).

A fuel sensor, wherein the intake terminal (4) is defined by a projecting end of cylindrical support (14), and wherein output terminal (5) is defined by a portion of the cover (11) projecting beyond the opposite end of the cylindrical support (14).

A fuel sensor, wherein the substrate plastic (20) supports and positions a temperature sensor (17).

A fuel sensor wherein each of said tabs (3) has through-holes, inside which is an oblong bushing (19), each of which preferably arrayed 90° relative to the other.

A fuel sensor, comprising a lid (7) to close the casing (2), said lid (7) being attached using a welding process such as to ensure the casing is hermetically sealed (2).

## Claims

1. A fuel sensor, specifically to detect the percentage ethanol in a fuel mixture comprised of gasoline and ethanol, said fuel sensor (1) comprising a paralleleliped casing (2) that internally defines a seat (9) for a circuit board PCB (8), wherein said casing (2) has external tabs (3) projecting from opposite sides, an electrical connection terminal (6), and two hydraulic connection terminals (4, 5) opposite each other and to be connected to a tube coming from the fuel reservoir and to a tube going to the fuel rail respectively, said fuel sensor (1) further comprising a sensing element (10) in the form of an external cylindrical cover (11) that goes around an internal cylindrical core (12), wherein said sensing element (10) is attached to casing (2) from a cylindrical support (14); sensing element (10) cover(11) is electrically connected to PCB board (8) from a first contact pin (15), the core (12) being electrically connected to PCB board (8) from a second contact pin (16), said contact pins (15, 16) passing through the cylindrical support (14) and supported and held in position by a plastic substrate (20) fixed to the interior of seat (9) of the casing (2).

2. The fuel sensor according to claim 1, wherein the first contact pin (15) comprises a cylindrical body provided with a first cylindrical upper projection (15') said first upper projection (15') being of a smaller diameter and able to penetrate a through-hole of the plastic substrate (20) and a respective through-hole of PCB board (8) and being welded thereto, and wherein the lower part of the cylindrical body of the first contact pin (15) is mechanically and electrically connected to the respective cover (11) of sensing element (10).

3. The fuel sensor according to claim 1, wherein the second contact pin (16) comprises a cylindrical body provided with a second upper cylindrical projection (16'), said second upper projection (16') being of a smaller diameter and able to penetrate a through-hole of the plastic substrate (20) and a respective through-hole of PCB board (8) and being welded thereto, and wherein the lower part of the cylindrical body of the second contact pin (16) is mechanically and electrically connected to said core (12) of the sensing element (10).

4. The fuel sensor according to any of the claims 1 to 3, wherein each of the first and second contact pins (15, 16) comprises respective sealing rings (18) or O-rings going around the cylindrical body of each of said contact pins.

5. The fuel sensor according to claim 1, wherein cylindrical support (14) is overinjected around the sensing element (10).

6. The fuel sensor according to claim 1 or 5, wherein the relative attachment between the cover (11) and core (12) is performed in the central region of the sensing element (10) with a lock (13), working with contact pins (15, 16).

7. The fuel sensor according to claim 1, wherein intake terminal (4) is defined by a projection from cylindrical support (14), wherein output terminal (5) is defined by a portion of cover (11) projecting beyond the opposite end of the cylindrical support (14).

8. The fuel sensor according to claim 1, wherein the plastic substrate (20) supports and positions a temperature sensor (17).

9. The fuel sensor according to claim 1, wherein each of said tabs (3) comprise through-holes, inside of which is an oblong bushing (19), each of which preferably arrayed 90° relative to the other.

10. The fuel sensor according to claim 1, wherein it comprises a lid (7) to close casing (2), said lid (7) being attached using a welding process such as to ensure the casing is hermetically sealed (2).
